# EUROPÄISCHE PATENTSCHRIFT

(11) **EP 1 137 378 B1**
(45) Veröffentlichungstag und Bekanntmachung des Hinweises auf die Patenterteilung: **27.08.2003**
(21) Anmeldenummer: 99952215.4
(22) Anmeldetag: 11.11.1999
(51) Int. Cl.: A61F 5/01, A61H 3/00

(54) **VORRICHTUNG UND VERFAHREN ZUR AUTOMATISIERUNG DER LAUFBANDTHERAPIE**
DEVICE AND METHOD FOR AUTOMATING TREADMILL THERAPY
DISPOSITIF ET PROCEDE D'AUTOMATISATION DE LA REEDUCATION A LA MARCHE SUR TAPIS ROULANT

(30) Priorität: 13.11.1998 CH 228598
(43) Veröffentlichungstag der Anmeldung: 04.10.2001
(73) Patentinhaber: Hocoma AG, 8712 Stäfa (CH)
(72) Erfinder: COLOMBO, Gery, CH-8006 Zürich (CH); JÖRG, Matthias, CH-8712 Stäfa (CH); HOSTETTLER, Peter, CH-4104 Oberwil (CH)
(74) Vertreter: Liebetanz, Michael, Dipl.-Phys.
(86) Internationale Anmeldenummer: CH9900531
(87) Internationale Veröffentlichungsnummer: WO00028927

(56) Entgegenhaltungen:
- EP-A- 0 782 843
- WO-A-96/09094
- DE-B- 1 055 177
- GB-A- 2 231 500
- US-A- 5 476 441

## Beschreibung

Die Erfindung betrifft eine Vorrichtung und ein Verfahren zur Automatisierung der Laufbandtherapie für die Rehabilitation von gehbehinderten Patienten gemäss Patentanspruch 1, bzw. 13.

Beim Laufbandtraining werden Patienten (z.B. inkomplett querschnittgelähmte Patienten oder Patienten nach einem Hirnschlag) auf einem Laufband trainiert. Sie werden zu diesem Zweck an einer speziellen Aufhängevorrichtung über dem Laufband aufgehängt. So können sie einerseits zu einem Teil von ihrem Körpergewicht entlastet werden und müssen sich andererseits nur noch wenig um die Aufrechterhaltung des Gleichgewichtes kümmern. Speziell in der ersten Zeit nach der Verletzung kann der Patient oft seine Beine gar nicht selber bewegen. Physiotherapeuten müssen deshalb die Beine der Patienten führen. Wird bei den Patienten möglichst früh nach der Verletzung ein intensives Training durchgeführt, so werden spezielle Bewegungszentren im Rückenmark des Patienten wieder trainiert und der Patient lernt besser gehen als dies mit konventionellen Therapieformen möglich ist. Dieses Führen der Beine ist eine sehr anstrengende Arbeit für die Therapeuten und sie ermüden bei der Ausführung dieses Trainings relativ schnell. Die Trainingseinheiten sind deshalb oft zu kurz und die Erfolge der Therapie nicht optimal.
Mit der hier beschriebenen Maschine kann dieses Training automatisiert werden. Es ist eine an Knie- und Hüftgelenken angetriebene Orthese, die an beliebige Patienten anpassbar ist. So werden während dem Training die Beine des Patienten in einem physiologischen Bewegungsablauf von der Orthese geführt. Im Gegensatz zur manuell geführten Therapie können bei der automatisierten Therapie grössere Erfolge erzielt werden, weil die Trainingseinheiten beliebig lange durchgeführt werden können. Es ist möglich, sehr früh nach der Verletzung des Patienten ein intensives Training durchzuführen. Bei einer Therapie mit der angetriebenen Orthese ist zudem nur ein Therapeut zur Betreuung des Patienten notwendig, das heisst es kann Personal eingespart werden.

Die Laufbandtherapie hat sich bei der Therapie von Patienten mit neurologischen Erkrankungen bereits in vielen Bereichen durchgesetzt. Sie wird speziell in Paraplegikerzentren häufig eingesetzt und ist in diesem Zusammenhang wissenschaftlich bestätigt. Die Therapie erfolgt zurzeit auf einem Laufband, wo die Patienten mit einem Gurt aufgehängt und die Beine von zwei Physiotherapeuten geführt werden (I. Wickelgren, Teaching the spinal cord to walk, Science, 1998, Vol. 279, 319-321). Mit Hilfe eines regelmässigen Laufbandtrainings können inkomplett querschnittgelähmte Patienten und Patienten nach einem Schlaganfall das Gehen viel schneller und besser wieder erlernen.
In der Rehabilitation von Patienten mit Bewegungseinschränkungen der Beine sind bereits verschiedene Orthesen im Einsatz. Passive Gangorthesen, wie z.B. in Patent US 5320590 (1994) beschrieben, werden bei der Rehabilitation von Querschnittgelähmten bereits regelmässig eingesetzt. Es existieren bereits einige Ansätze für angetriebene Orthesen. Die Patente US 5020790 (1991) und GB 2260495 (1991) beschreiben solche, bei welchen Knie- und Hüftgelenke mit hydraulischen Zylindern oder Elektromotoren bewegt werden. Mit diesen Orthesen ist es möglich, einem Patienten, für welchen die jeweilige Orthese speziell angefertigt wurde, die Beine in einem dem Gehen ähnlichen Bewegungsmuster zu bewegen.
In der Patentanmeldung EP 0782843 A2 (1996) wird eine Orthese beschrieben, welche ebenfalls an den Knie- und Hüftgelenken angetrieben wird. Der Patient absolviert sein Training allerdings auf einem Laufband. Die Kontrolle der Beinbewegungen erfolgt über Schalter, welche der Patient während dem Gehen manuell betätigt und welche jeweils eine Streckung respektive eine Biegung des Beines bewirken.

Es ist die Aufgabe der vorliegenden Erfindung, das bisher manuell durchgeführte Laufbandtraining an Patienten in der Rehabilitation zu automatisieren.

Erfindungsgemäss wird diese Aufgabe mit einer angetriebenen Orthese gemäss dem Wortlaut des Patentanspruches 1 und einem dazugehörigen Verfahren zum Betrieb der Orthese gemäss dem Wortlaut des Patentanspruches 13 gelöst.

Es geht darum, die Beine des über dem Laufband positionierten Patienten in einem möglichst physiologischen Gangmuster zu führen. Dabei wird eine Regelung der Antriebe benötigt, welche diese Antriebe gemäss vorgegebenem Bewegungsablauf regelt.
Der Patient soll so auf dem Laufband stabilisiert werden, dass er sich nicht um die Stabilität seines Gleichgewichtes kümmern muss. Somit kann er sich auf ein dynamisches, physiologisches Gangmuster konzentrieren.
Die Orthese soll in den Rehabilitationszentren für das Training von verschiedenen Patienten einsetzbar sein und muss deshalb in Grösse und Form verstellbar sein.
Die Orthese muss so konzipiert sein, dass am Körper des Patienten keine Druckstellen entstehen können, da speziell Paraplegiker schnell Druckgeschwüre kriegen.

Die Erfindung wird im Folgenden anhand der Zeichnungen näher erläutert. Es zeigen:
- Fig. 1: Prinzip des Laufbandtrainings mit angetriebener Orthese in schematischer Darstellung, Fixierung mit Parallelogramm
- Fig. 2: Parallelogramm zur Fixierung der angetriebenen Orthese auf dem Laufband
- Fig. 3: Prinzip des Laufbandtrainings mit angetriebener Orthese in schematischer Darstellung, Fixierung mit Rollenführung
- Fig. 4: Rollenführung zur Fixierung der angetriebenen Orthese auf dem Laufband
- Fig. 5: Gesamtansicht der angetriebenen Orthese
- Fig. 6: Ansicht der verstellbaren Hüftorthese
- Fig. 7: Ausführungsbeispiel eines Knieantriebes mit Kugelgewindespindel
- Fig. 8: Ausführungsbeispiel einer Manschette zur Fixierung der Beine
- Fig. 9: Übersicht über die Regelung des Therapiesystems
- Fig. 10: Regeleinheit

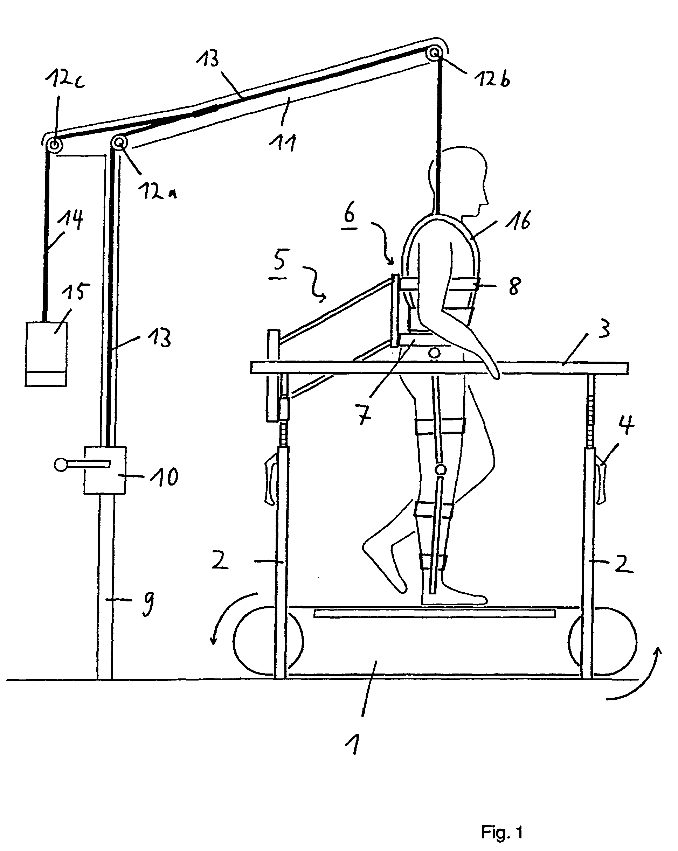

Fig. 1 zeigt das Prinzip des Laufbandtrainingssystems mit angetriebener Orthese in schematischer Darstellung, in der Variante mit Parallelogramm für die Stabilisierung des Patienten. An einem Laufband 1 ist auf jeder Seite der Lauffläche auf Stützen 2 je ein Geländer 3 montiert, welches sich wie bei einem Gehbarren mit einem Mechanismus 4 in der Höhe verstellen lässt. Am hinteren Ende des Geländers 3 ist ein Parallelogramm 5 beweglich befestigt, das später näher beschrieben wird. Das Parallelogramm 5 dient zur Stabilisierung einer Orthese 6, die zur Aufnahme des Patienten vorgesehen ist und die sich über dem Laufband 1 befindet. Das Parallelogramm 5 lässt eine Bewegung der Orthese 6 nur auf einem vorgegebenen Kreissektor zu, wobei die Bewegung mit einem Pfeil angedeutet ist. Dadurch wird die Orthese 6 und damit auch der Patient stabilisiert, so dass er weder seitlich noch vorund rückwärts kippen kann. Der Oberkörper des Patienten wird durch einen Hüftgurt 7 und einen Brustgurt 8 mit der Orthese 6 verbunden und so in einer konstanten Position zur Vertikalen gehalten. Durch die Höhenverstellbarkeit des Geländers 3 kann auch die Höhe des Parallelogramms 5 verstellt werden. Das Parallelogramm 5 wird bei verschieden grossen Patienten bei der Anpassung des Geländers 3 automatisch ebenfalls in der Höhe angepasst.
Hinter dem Laufband 1 ist weiter eine Aufhängevorrichtung angebracht, die aus einer Stütze 9, einer Seilwinde 10, einem Auslegearm 11, Rollen 12a, 12b und 12c, Drahtseilen 13 und 14 und einem variablen Gegengewicht 15 besteht. Von der Seilwinde 10 führt das Drahtseil 13 über die Rollen 12a und 12b zum Patienten. Dieser trägt einen Laufbandgurt 16, an welchem das Drahtseil 11 befestigt wird. Am zwischen den Rollen 12a und 12b liegenden Teil des Drahtseils 13 wird ein zweites Drahtseil 14 befestigt, das über die Rolle 12c geführt wird und an dessen Ende das Gegengewicht 15 eingehängt werden kann. Bei Therapiebeginn wird dem im Rollstuhl sitzenden Patienten der Laufbandgurt 16, der in bekannter Weise bei der manuellen Laufbandtherapie eingesetzt wird, angezogen. Der Laufbandgurt 16 wird dann mit einem Karabiner an das Drahtseil 13 gehängt und der Patient mit der Seilwinde 10 nach oben gezogen. Wenn der Patient sich in einer aufrechten Lage befindet, wird das Gegengewicht 15 angehängt, wodurch der Patient bei der Therapie teilweise von seinem Körpergewicht entlastet wird. Mit der Seilwinde 10 kann danach das Drahtseil 13 wieder leicht gelöst werden, wonach der Patient mit konstanter Entlastung auf dem Laufband gehen kann. Das Gegengewicht 15 wird im Verlauf der Rehabilitation verringert und so die Belastung der Beine immer mehr erhöht, bis die Beine des Patienten sein ganzes Gewicht tragen können.

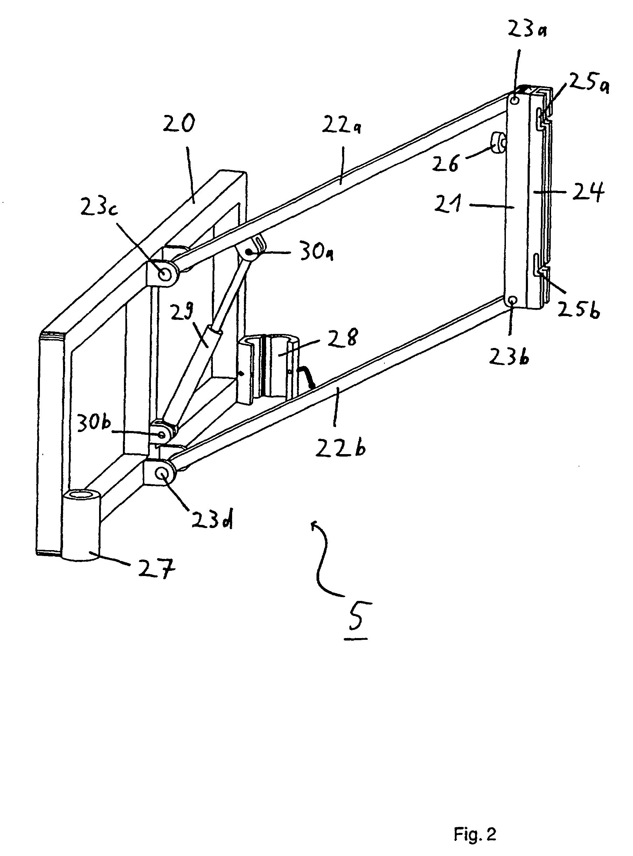

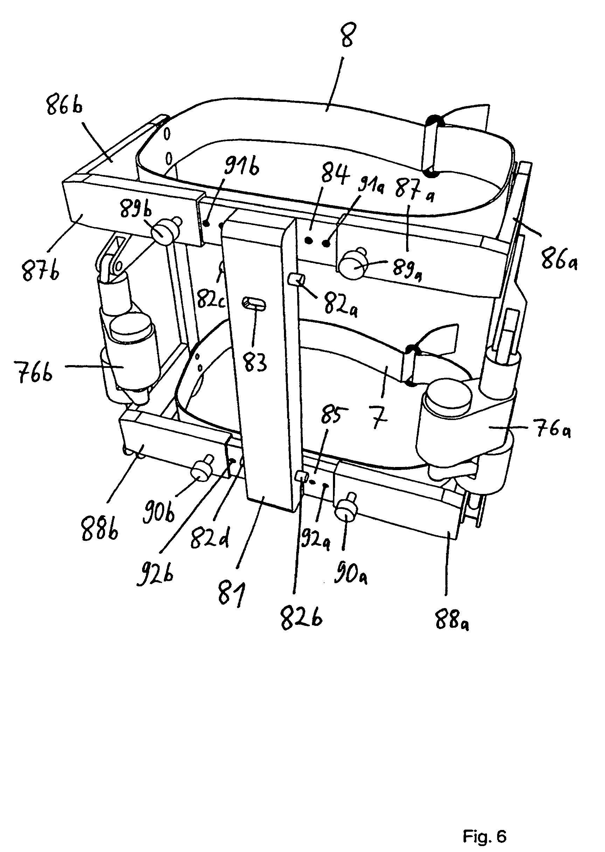

Fig. 2 zeigt das Parallelogramm 5 zur Fixierung der angetriebenen Orthese auf dem Laufband. Das Parallelogramm besteht aus einem rahmenförmigen Grundgestell 20, einem Orthesenteil 21, zwei Trägern 22a und 22b, die das Grundgestell 20 mit dem Orthesenteil 21 verbinden, und einem Orthesenhalter 24. Die beiden Träger 22a und 22b sind an den Lagern 23a - 23d so gelagert, dass sich der Orthesenteil 21 nur parallel zum Grundgestell 20 bewegen kann. Am Orthesenteil 21 befindet sich an der Aussenseite zum Parallelogramm der Orthesenhalter 24, ein U-förmiges Profil, mit Führungen 25a und 25b, die als L-förmige Schlitze ausgebildet sind. In diese Schlitze wird die Orthese eingehängt und mit einem Nocken 26 fixiert (siehe auch Fig. 6). Durch Herausziehen des Nockens 26, kann die Orthese wieder gelöst und ausgehängt werden.
Das Parallelogramm 5 ist mittels einem Lager 27, das am unteren Teil des Grundgestells 20 angebracht ist, an einem Geländer des Laufbandes so befestigt, dass es sich horizontal frei drehen lässt. So kann das Parallelogramm von aussen über das Laufband gedreht und mit einem Verschluss 28, der auf der dem Lager 27 gegenüberliegenden Seite des unteren Teils des Grundgestells 20 angebracht ist, auf der anderen Seite des Laufbandes wiederum am anderen Geländer befestigt werden. Dadurch kann ein Patient mit seinem Rollstuhl auf das Laufband fahren, bzw. gefahren werden, wenn das Parallelogramm im Zustand "geöffnet" ist. Wenn der Patient mit der Aufhängevorrichtung aus seinem Rollstuhl gehoben und der Rollstuhl vom Laufband weggefahren wurde, kann das Parallelogramm "geschlossen" werden. Die Orthese, welche am Orthesenhalter 24 befestigt ist, kann dann dem Patienten angepasst und an ihm fixiert werden. Zwischen dem Grundgestell 20 und dem oberen Träger 22a ist eine Gasdruckfeder 29 an Lagern 30a und 30b befestigt, welche das Gewicht der Orthese und des Parallelogramms kompensiert, indem sie den Träger 22a mit derjenigen Kraft nach oben drückt, die notwendig ist, um die Orthese quasi schwerelos am Parallelogramm 5 zu bewegen. Anstelle der Gasdruckfeder 29 kann an der selben
Stelle eine mechanische Feder plaziert werden, um das Gewicht der Orthese zu kompensieren.
Eine weitere Möglichkeit (nicht dargestellt), die Orthese teilweise von ihrem Gewicht zu entlasten, besteht darin, über dem Grundgestell 20 eine Rolle anzubringen über welche ein Drahtseil geführt wird, welches beim Lager 30a befestigt und auf der anderen Seite des Parallelogramms mit einem Gegengewicht beschwert ist.

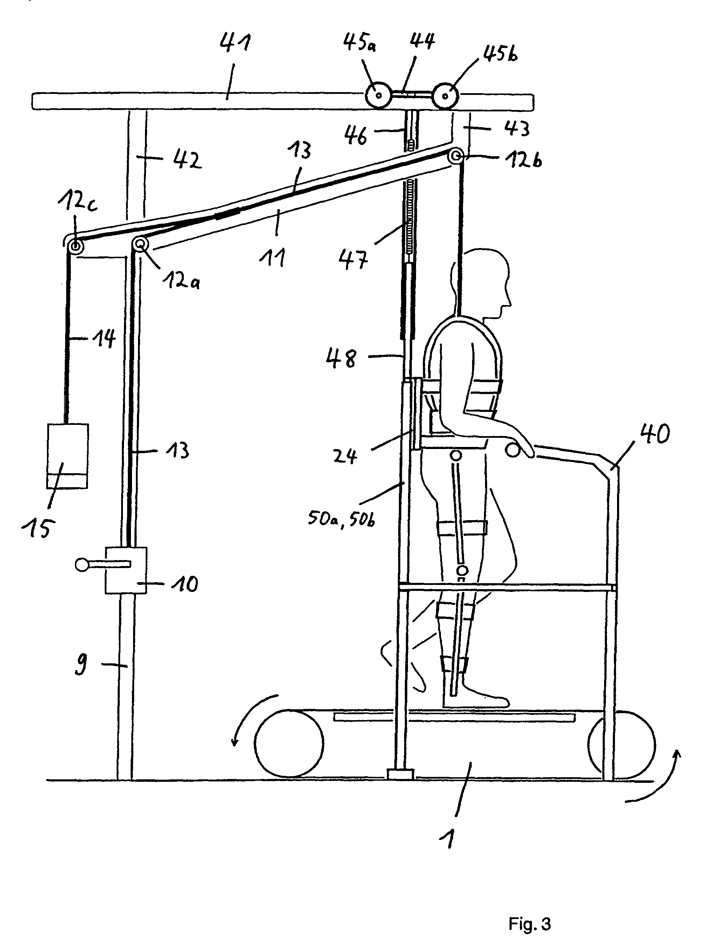

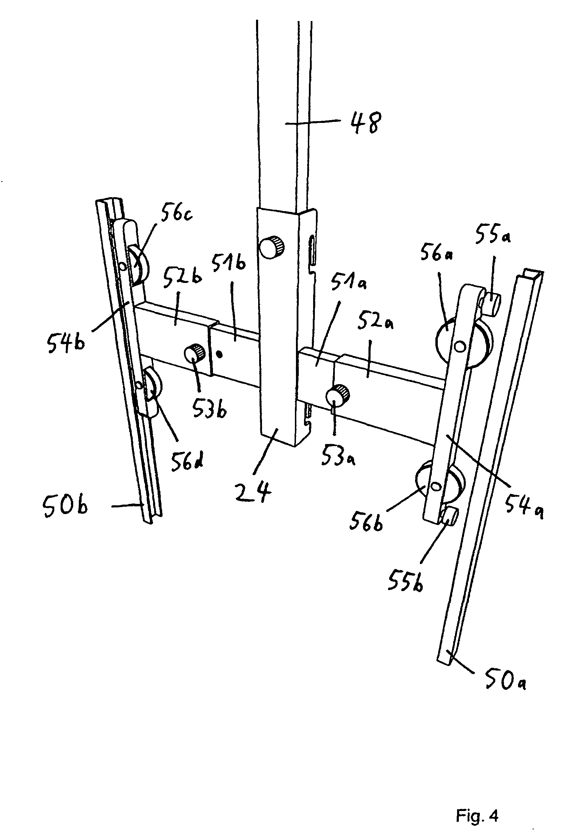

Fig. 3 zeigt das Prinzip des Laufbandtrainingssystems mit angetriebener Orthese in schematischer Darstellung, in der Variante mit einer Rollenführung zur Stabilisierung des Patienten. Dies ist eine Alternative zu der in Fig. 1 beschriebenen Stabilisierung, wobei die in Fig. 1 beschriebene Lösung für den Einsatz mit der angetriebenen Orthese zu bevorzugen ist.
Am Laufband 1 ist eine andere Variante eines Geländers 40 montiert, welches in der Höhe verstellt werden kann. Wie bei der Variante mit Parallelogramm ist hinter dem Laufband 1 die Aufhängevorrichtung angebracht, die aus der Stütze 9, der Seilwinde 10, dem Auslegearm 11, den Rollen 12a, 12b und 12c, den Drahtseilen 13 und 14 und dem Gegengewicht 15 besteht.
Eine Schiene 41, ist mit Trägern 42 und 43 auf der Stütze 9 resp. dem Auslegearm 11, über dem Laufband 1 angebracht. Ein Wagen 44, mit zwei Rollen 45a und 45b befindet sich auf der Schiene 41 und kann auf dieser vor- und zurückfahren. Senkrecht nach unten ist am Wagen 44 ein Führungsrohr 46 (Vierkantrohr) angebracht, in welchem sich eine Feder 47 befindet, welche am oberen Ende des Führungsrohres 46 befestigt ist. Diese Feder zieht mit einer Kraft nach oben, die das Gewicht der Orthese kompensiert. Am unteren Ende der Feder 47 ist ein Vierkantrohr 48, welches in das Führungsrohr 46 passt und so von diesem geführt wird. Am unteren Ende des Vierkantrohrs 48 sind der Orthesenhalter 24 und eine Rollenführung (siehe Fig. 4) befestigt. Auf jeder Seite des Laufbandes 1 ist je eine Führungsschiene 50a resp. 50b befestigt.

Fig. 4 zeigt die Rollenführung zur Stabilisierung der angetriebenen Orthese auf dem Laufband. Am unteren Ende des Vierkantrohres 48 erkennt man den Orthesenhalter 24, welcher auf jeder Seite wiederum ein senkrecht zum Orthesenhalter 24 angebrachtes Vierkantrohr 51 a und 51 b aufweist. Zwei weitere Vierkantrohre 52a und 52b, welche die Rohre 51 a und 51 b umschliessen, können mit Rastermechanismen 53a und 53b in zwei verschiedenen Positionen an den Rohren 51a und 51b fixiert werden. In Fig. 4 wird die linke Seite im 'ausgefahrenen' und die rechte im 'eingefahrenen' Zustand gezeigt. An den äusseren Enden der Rohre 52a und 52b sind Rollenhalter 54a und 54b angebracht, an welchen wiederum Rollen 55a - 55d und 56a - 56d befestigt sind. Die Rollen 55c und 55d sind auf der Figur nicht erkennbar. Im ausgefahrenen Zustand der Rohre 52a und 52b, laufen die Rollen 55a - 55d sowie 56a - 56d in den zwei Führungsschienen 50a und 50b. Die Rollen 55a - 55d sorgen dafür, dass die Orthese mit dem Patienten nicht vor- oder rückwärts kippen kann, die Rollen 56a - 56d sorgen für die seitliche Stabilität.
Bei einer Therapie mit dem Laufbandtrainingssystem mit angetriebener Orthese und mit einer Rollenführung dieser Art zur Stabilisierung des Patienten, wird der Patient - wie in Fig. 2 beschrieben - über dem Laufband aufgehängt. Die Orthese wird dann von hinten, über den Wagen geführt, an den Patienten geschoben und angepasst. Dann werden die Rohre 52a und 52b in die Führungsschienen 50a und 50b ausgefahren, um den Patienten mittels den Rollen 55a - 55d, sowie den Rollen 56a - 56d zu stabilisieren.

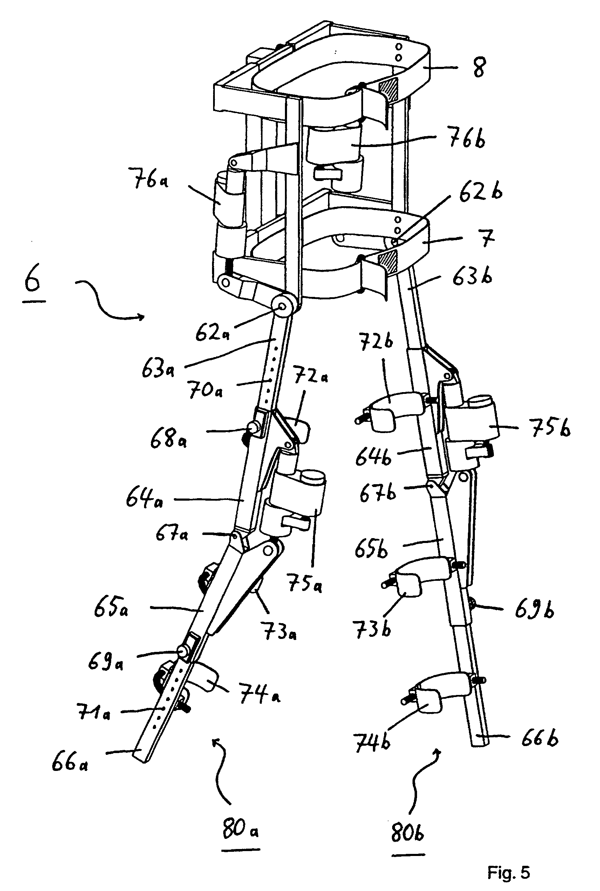

Fig. 5 zeigt eine Übersicht über die angetriebene Orthese 6. Sie besteht im wesentlichen aus einer Hüftorthese (siehe Fig. 6) und zwei Beinteilen 80a und 80b. Die Hüftorthese ist eine in der Breite verstellbare und an den Patienten anpassbare Orthese, in welcher der Oberkörper des Patienten mit dem Hüftgurt 7 und dem Brustgurt 8 fixiert wird. Bei den Gurten 7 und 8 handelt es sich um relativ breite Klettbänder mit je einem Verschluss auf der hinteren und der vorderen Seite. Auf jeder Seite der Hüftorthese sind am unteren Teil je ein Kugellager 62a resp. 62b angebracht. An diesen sind die beiden Beinschienen 63a und 63b beweglich befestigt. Diese Lager führen die Beinschienen 80a und 80b beim Gehen auf dem Laufband in einer Ebene parallel zur Bewegungsebene der Beine des Patienten. Die Hüftorthese muss dem Patienten so angepasst werden, dass die Hüftgelenke des Patienten direkt unter das Lager 62a, resp. 62b zu liegen kommen.
Die Beinschienen 63a, 63b, 64a, 64b, 65a, 65b, 66a und 66b der Beinteile 80a und 80b sind als Vierkantrohre ausgebildet. Die Rohre 64a, 65a und 64b, 65b umschliessen die Rohre 63a, 66a und 63b, 66b und sind über die Kniegelenke 67a und 67b miteinander verbunden. Die Rohre 63a, 63b und 64a, 64b sowie 65a, 65b und 66a, 66b lassen sich ineinander verschieben. Die Gleitflächen zwischen den ineinander geschobenen Rohren ermöglichen eine mühelose und dennoch praktisch spielfreie Verstellbarkeit der Beinlängen. An einem Ende der Rohre 64a, 64b und 65a, 65b ist je ein Rastermechanismus 68a, 68b (nicht sichtbar) und 69a, 69b angebracht, welcher diese Rohre mit einem Bolzen in den in regelmässigen Abständen in den Rohren 63a, 63b und 66a, 66b angebrachten Lochreihen 70a, 70b und 71a, 71b (beide nicht sichtbar) fixiert. So können die Längen der Beinteile 80a und 80b jeweils auf die Schenkellängen des Patienten angepasst und die Lage der Gelenke 67a und 67b mit den Kniegelenken des Patienten in Übereinstimmung gebracht werden. Die Löcher sind jeweils durchnummeriert, um ein Ablesen der Grösseneinstellung zu ermöglichen, was für eine rasche Anpassung bei mehrmaliger Therapie eines Patienten wesentlich ist.
In den Hüft- und Kniegelenken 62a, 62b resp. 67a, 67b sind Winkelsensoren (Potentiometer) integriert, welche für die Regelung der Orthese verwendet werden. Die Beinschienen 80a und 80b werden mittels Manschetten 72a, 72b, 73a, 73b und 74a, 74b an den Beinen des Patienten befestigt. Das Manschettenpaar 72a, 72b ist schienenseitig an den Rohren 64a, 64b, das Manschettenpaar 73a, 73b an den Rohren 65a, 65b und das Manschettenpaar 74a, 74b an den Rohren 66a, 66b befestigt.
Zur Bewegung der Knie-, resp. Hüftgelenke sind Knieantriebe 75a, 75b resp. Hüftantriebe 76a, 76b vorgesehen.
Die Beinschienen 64a und 64b können aus den Beinschienen 63a und 63b ganz herausgezogen werden. Die Kabel bzw. elektrischen Zuführungen der Sensoren und Aktoren unterhalb der Schienen 63a resp. 63b sind mit einem Steckverbinder abtrennbar. So kann jedes Beinteil einzeln von der angetriebenen Orthese 6 entfernt werden. Dies ermöglicht es, bei Patienten mit Hemiparese nur ein Bein aktiv zu führen und ein allfällig gesundes Bein selbständig gehen zu lassen (Ein-Bein-Therapie).

Fig. 6 zeigt schematisch eine in der Breite verstellbare Hüftorthese. Ein Orthesenrückenteil 81, hat auf jeder Seite zwei Bolzen 82a, 82b, sowie 82c, 82d eingelassen, die es erlauben, die Hüftorthese in den Führungen des Orthesenhalters einzuhängen. Auf der Rückseite des Orthesenrückenteils 81 ist ein Arretierungsloch 83, in welchem der Nocken des Orthesenhalters einschnappen kann, um die Hüftorthese im Orthesenhalter zu fixieren. Auf der vorderen Seite des Orthesenrückenteils 81 sind zwei vierkantige Rohre 84 und 85 angebracht. An zwei Hüftseitenteilen 86a und 86b sind oben und unten ebenfalls vierkantige Rohre 87a und 87b sowie 88a und 88b angebracht, welche die Rohre 84 und 85 je auf einer Seite des Orthesenrückenteils umschliessen und auf diesen frei verschiebbar sind. Mit den Rastermechanismen 89a und 89b sowie 90a und 90b, welche je mit einem Nocken in die Lochreihen 91a und 91b sowie 92a und 92b eingerastet werden können, werden die Hüftseitenteile 86 a und 86b in der korrekten Position fixiert. So kann durch ein Verschieben der Hüftseitenteile 86a und 86b die Hüftorthese auf die individuellen Anforderungen (Hüftbreite) der Patienten eingestellt werden. Bedingt durch die starre Verbindung der Rohre 87a und 88a resp. 87c und 88b durch die Hüftseitenteile 86a und 86b, müssen die Rastermechanismen 89a und 90a resp. 89b und 90b jeweils gleichzeitig gelöst werden um einen Hüftseitenteil zu verschieben. Auf der Innenseite der Hüftseitenteile 86a und 86b sind jeweils oben innen der Hüftgurt 7 und unten innen der Brustgurt 8 befestigt. Auf der Figur ebenfalls erkennbar sind die zwei Hüftantriebe 76a und 76b.

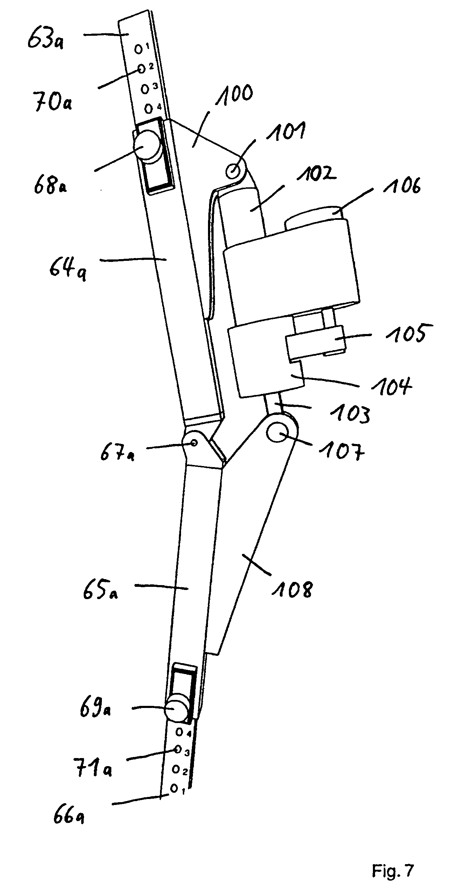

Fig. 7 zeigt ein Ausführungsbeispiel eines Antriebes mit Kugelgewindespindel für das rechte Kniegelenk. An der Beinschiene 64a ist eine Halterung 100 angebracht. An dieser Halterung 100 befindet sich ein Bolzen 101. Ein Führungszylinder 102 des Spindelantriebes ist über ein Rollenlager an diesem Bolzen 101 gelagert. In diesem Führungszylinder 102 bewegt sich eine Kugelgewindespindel 103, welche in dieser Darstellung beinahe ganz eingefahren ist. Im Gewindemuttergehäuse 104 befindet sich die Kugelgewindespindelmutter (nicht sichtbar), welche so im Gehäuse gelagert ist, dass sie über einen Zahnkeilriemen 105 vom einem Elektromotor 106 angetrieben werden kann. Der aus dem Führungszylinder 102 ausfahrende Teil der Kugelgewindespindel 103 ist mit einem Rollenlager an einem Bolzen 107 gelagert, der in einer Halterung 108 befestigt ist. Die Halterung 108 wiederum ist fest mit der Beinschiene 65a verbunden.
Die Beinschiene 64a ist mit der Beinschiene 65a über das Kugellager 67a gelenkig verbunden. Wenn der Elektromotor 106 über den Zahnkeilriemen 105 die Spindelmutter dreht, so bewegt sich die Kugelgewindespindel 103 ("stehende Spindel") in den Führungszylinder 102 hinein resp. aus ihm heraus. Dies ergibt eine Extension (Streckung) resp. eine Flexion (Biegung) der Beinorthese um das Lager 67a.
Am oberen Ende der Beinschiene 64a, bzw. am unteren Ende der Beinschiene 65a sind die Rastermechanismen 68a bzw. 69a erkennbar, sowie die in den Rohren 63a bzw. 66a angebrachten nummerierten Lochreihen 70a resp. 71 a, welche der raschen Anpassung der Länge der Beinteile an den Patienten dienen. Die Lochreihen 70a und 71a sind mit Markierungen (Nummern) versehen, damit die Einstellungen an diesen abgelesen werden können. Die Einstellungen werden benötigt, um einen Regler zu konfigurieren. Sie können aber auch gespeichert werden, um bei einem späteren Training die Orthese für den Patienten wieder richtig einzustellen.
Vom Prinzip her identisch werden mit demselben Kugelgewindespindelantrieb das linke Knie und die beiden Hüftgelenke angetrieben.

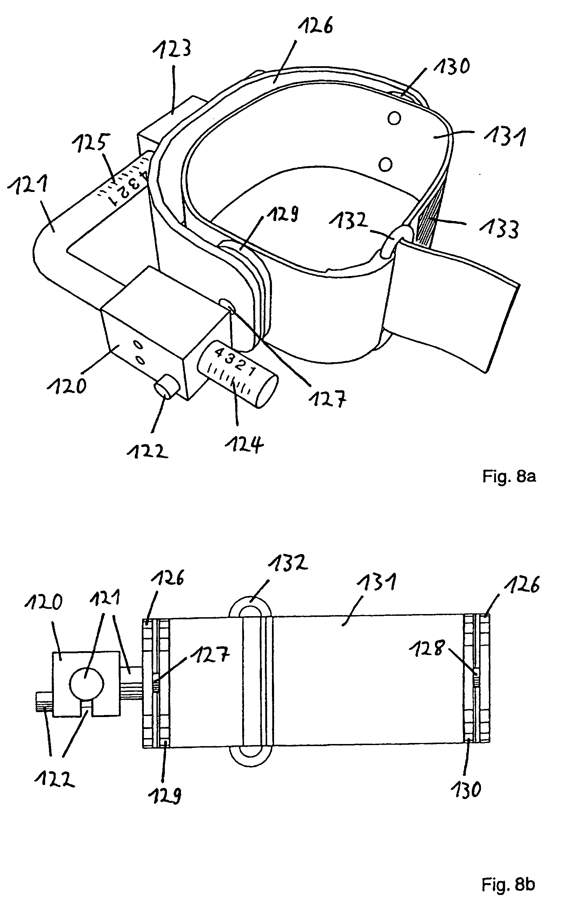

Fig. 8a und 8b zeigen eine Manschette zur Fixierung der Beine: Fig. 8a ist eine perspektivische Darstellung, Fig. 8b eine Ansicht von vorne. Ein Haltemechanismus 120, von welchem je einer an den Beinschienen befestigt ist, weist eine runde Öffnung auf, in die ein Metallrohr 121 geführt wird. Das Metallrohr 121 kann in dem Haltemechanismus 120 frei verschoben werden und an der richtigen Position mit einem Schnellschraubmechanismus 122 festgemacht werden. Auf dem anderen Schenkel des rechtwinklig gebogenen Metallrohres 121 befindet sich ein zweiter Haltemechanismus 123, identisch zum Haltemechanismus 120, welcher gleich wie dieser frei auf dem Rohr verschoben und in der richtigen Position fixiert werden kann. So können die Beine des Patienten optimal in der Orthese positioniert werden, damit die Bewegungsebenen sowie die Gelenkpositionen der Orthese und der Beine des Patienten übereinstimmen. Die Positionen, an welchen die Halterungen 120 und 123 auf dem Rohr fixiert werden, sind mit Markierungen 124 und 125 gekennzeichnet, damit die Einstellungen jederzeit rekonstruiert werden können.
An den Haltemechanismus 123 ist ein steifer, halbrunder Bügel 126 geschraubt. An den Enden dieses Bügels 126 ist je ein Lager 127 und 128 befestigt. In diesen Lagern können sich je ein Metallplättchen 129 und 130 frei drehen. An der Innenseite der Metallplättchen ist ein Klettband 131 so angebracht, dass der hintere, bügelseitige Teil des Bandes geschlossen und der vordere offen ist. Der hintere Teil ist so lange gewählt, dass das Bein eines Patienten in der Manschette Platz findet, das Band aber im gespannten Zustand (wenn ein Bein darin fixiert ist) den Bügel 126 nicht berührt. Am vorderen, offenen Teil des Bandes 131 ist am einen Ende ein Metallbügel 132 angebracht, durch welchen das andere Ende des Bandes eingeschlauft werden kann. So kann das Band straff um das Bein des Patienten angezogen und mit einem Klettverschluss 133 fixiert werden. Diese drehbar gelagerten Bänder erlauben es, die Kräfte, welche auftreten, wenn die Beinorthese die Beine des Patienten bewegt, möglichst gleichmässig auf die Haut des Patienten zu verteilen. Das Bein des Patienten kommt nicht in Berührung mit festen Teilen der Orthese. Dies ist wichtig, damit keine Druckgeschwüre entstehen.

Besonders vorteilhaft erweist sich, dass bei der ersten Anpassung der Orthese an einen Patienten sämtliche Einstellungen an Markierungen abgelesen werden, dass die so ermittelten Werte gespeichert werden und dass bei einer späteren Therapie mit demselben Patienten die Orthese gemäss diesen Werten wieder eingestellt wird.

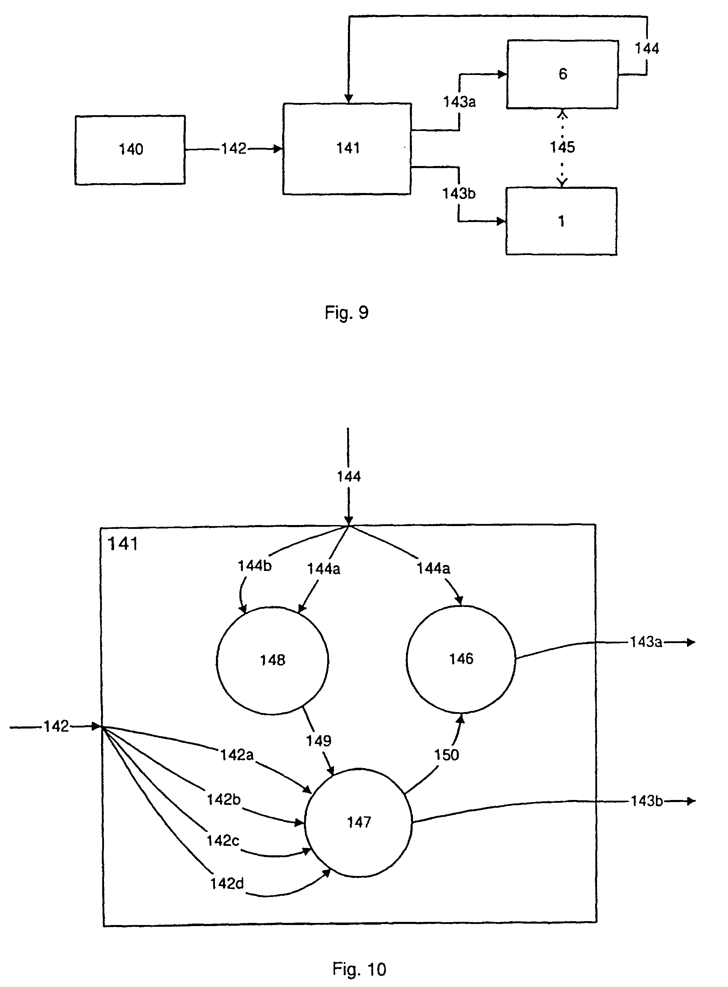

Fig. 9 zeigt eine Regelung des Therapiesystems in der Übersicht. Die Regelung besteht aus einem Eingabegerät 140, einer Regeleinheit 141, dem Laufband 1 und der angetriebenen Orthese. Über das Eingabegerät 140 kann die Regeleinheit 141 über Benutzerdaten 142 konfiguriert werden. Die Regeleinheit 141 dient der Erzeugung und Regelung natürlicher, d.h. physiologischer Gangmuster an der Orthese 6 und der Steuerung des Laufbands 1. Die Orthese erhält als Steuersignale 143a Positions-Sollwerte für die Knie- und Hüftantriebe. Die Messwerte der in den Knie- und Hüftgelenken integrierten Winkelsensoren werden in die Regeleinheit 141 als Messgrössen 144 zurückgeführt, wodurch ein Regelkreis (141 → 143a → 6 → 144 → 141) entsteht, über den die Beinposition der Orthese 6 exakt geregelt und mit der Laufbandgeschwindigkeit synchronisiert wird. Ein Steuersignal 143b führt zum Laufband 1 und ermöglicht die Steuerung der Laufbandgeschwindigkeit, welche am Eingabegerät 140 vorgegeben werden kann.
Eine zentrale Aufgabe der Regeleinheit 141 ist die Synchronisation des Bewegungsablaufs der Orthese 6 mit der Laufbandgeschwindigkeit: Aufgrund der an Knien und Hüfte angetriebenen Orthese 6 und dem in der Geschwindigkeit gesteuerten Laufband 1 entsteht jeweils während der Standphase eine Kopplung 145 zwischen Orthese 6 und Laufband 1, welche zu einer Überbestimmtheit des Systems führt, die durch eine Synchronisation der Beinbewegung mit der Laufbandgeschwindigkeit eliminiert wird.

Fig. 10 zeigt die Regeleinheit 141 im Detail. Die Schnittstellen der Regeleinheit 141 bestehen aus den Benutzerdaten 142, den Steuersignalen 143a und 143b und den Messgrössen 144. Die Benutzerdaten 142 umfassen eine Vorgabe der Schrittlänge 142a, eine Vorgabe der Laufbandgeschwindigkeit 142b, sowie sämtliche patientenspezifischen Einstellungen 142c, insbesondere die an der Orthese eingestellte Beinlänge. Zu den Benutzerdaten 142 gehören zudem Bewegungssollkurven 142d, durch welche ein zu erzeugendes Gangmuster 150 optimal an den Patienten angepasst werden kann. Die Steuersignale 143a sind mit den Knie- und Hüftantrieben der Orthese verbunden und geben die Sollposition von Knie- und Hüftgelenken vor. Das Steuersignal 143b erlaubt die Geschwindigkeitssteuerung des Laufbandes. Die Messgrössen 144 teilen sich auf in Signale 144a von Winkelsensoren, welche in die Knie- und Hüftgelenke der Orthese integriert sind, und in Signale 144b von Fussschaltern, welche in der Ganganalyse häufig verwendet werden und an bzw. in den Schuhen des Patienten angebracht sind.
Die Benutzerdaten 142 müssen zu Beginn einer Therapiesession eingegeben bzw. von einem Datenspeicher in die Regeleinheit 141 geladen werden. Eine Änderung der patientenspezifischen Einstellungen 142c und der Bewegungssollkurven 142d kann aus Sicherheitsgründen nur bei stillstehender Orthese vorgenommen werden. Die Schrittlänge 142a und die Laufbandgeschwindigkeit 142b können hingegen auch während dem Betrieb der angetriebenen Orthese verändert werden.
Die zentralen Prozesse der Regeleinheit 141 sind ein Positionsregler 146, ein Phasenregler 147 und eine Triggereinheit 148. Der Positionsregler 146 erzeugt die Steuersignale 143a, welche als Sollwerte für die Beinposition zu den Knie- und Hüftantrieben der Orthese geführt werden. Der Regelkreis des Positionsreglers 146 wird durch die Rückführung der Messgrössen 144a der Winkelsensoren der Orthese geschlossen, wodurch die Beinposition der Orthese exakt geregelt werden kann. Der Phasenregler 147 befindet sich zusammen mit der Triggereinheit 148 in einem dem Positionsregler 146 übergeordneten Regelkreis, welcher aufgrund der vorgegebenen Laufbandgeschwindigkeit 142b und der mechanischen Einstellungen der Orthese (patientenspezifische Einstellungen 142c) die Bewegungssollkurven 142d zeitlich so skaliert, dass die vorgegebene Schrittlänge 142a im Mittel möglichst genau erreicht wird und der Bewegungsablauf der Orthese mit der Laufbandgeschwindigkeit 142b synchronisiert wird: Bei einer vorgegebenen Schrittlänge 142a soll das Schwungbein des Patienten, d.h. dasjenige Bein, welches nicht auf dem Laufband steht, immer an der gleichen Position auf dem Laufband auftreten. Der Wechsel von Schwung- zu Standbein und umgekehrt wird dem Phasenregler 147 durch das von der Triggereinheit 148 stammende Triggersignal 149 angezeigt. Die Triggereinheit 148 bezieht die dazu notwendige Information aus den Messgrössen 144, d.h. aus den Signalen 144a der Beinposition der Orthese (Knie- und Hüftwinkel), sowie den Signalen 144b der Fussschalter. Das so im Phasenregler 147 berechnete Gangmuster 150 wird dem Positionsregler 146 zur Verfügung gestellt, dient als Vorgabe für die Beinposition und wird über die Steuersignale 143a an die Orthese weitergeleitet.

## Patentansprüche

1. Vorrichtung für das Laufbandtraining von gehbehinderten Patienten, umfassend ein Laufband, einen Entlastungsmechanismus für den Patienten und eine angetriebene Orthese, **gekennzeichnet dadurch, dass** Mittel zur Stabilisierung der Orthese (6) vorgesehen sind, die ein Kippen des Patienten nach vorne, nach hinten und nach der Seite verhindern, dass die Orthese (6) aus einer Hüftorthese und zwei Beinteilen (80a, 80b) besteht, wobei zur Bewegung der Hüftorthese zwei Hüftantriebe (76a, 76b) und zur Bewegung der Beinteile zwei Knieantriebe (75a, 75b) vorgesehen sind, dass Hüftorthese und Beinteile (80a, 80b) verstellbar sind, wobei die Beinteile (80a, 80b) in Grösse und Position verstellbare Manschetten (72a, 72b, 73a, 73b, 74a, 74b) aufweisen, und dass für die Regelung der Bewegungen der Orthese (6) und für die Steuerung der Geschwindigkeit des Laufbandes (1) eine Regeleinheit (141) vorgesehen ist.

2. Vorrichtung nach Anspruch 1, **dadurch gekennzeichnet, dass** als Mittel zur Stabilisierung der Orthese (6) ein Parallelogramm (5) vorgesehen ist, das am Geländer (3) des Laufbandes (1) höhenverstellbar fixiert ist.

3. Vorrichtung nach Anspruch 2, **dadurch gekennzeichnet, dass** das Parallelogramm (5) aus einem Grundgestell (20), einem Orthesenteil (21) und zwei Trägern (22a, 22b) besteht, die untereinander über Lager (23a - 23d) verbunden sind, dass am Grundgestell (20) auf der einen unteren Seite ein erstes Lagerelement (27) angebracht ist, mit welchem das Grundgestell (20), resp. das Parallelogramm (5), schwenkbar gelagert und am ersten Geländer (3) des Laufbandes (1) höhenverstellbar fixiert ist, dass am Grundgestell (20) auf der anderen unteren Seite ein zweites aufklappbares und verschliessbares Lagerelement (28) angebracht ist, mit welchem das Grundgestell (20), resp. das Parallelogramm (5), nach erfolgter Drehbewegung um das erste Lagerelement (27) am zweiten Geländer (3) des Laufbandes (1) arretiert werden kann, und dass am Orthesenteil (21) ein Orthesenhalter (24) angebracht ist, der Mittel zur Befestigung der Orthese (6) aufweist.

4. Vorrichtung nach Anspruch 2 oder 3, **dadurch gekennzeichnet, dass** am Parallelogramm (5) zur Kompensation des Gewichtes der Orthese (6) ein Entlastungsmechanismus angebracht ist, wobei hierfür vorzugsweise eine Gasdruckfeder (29), ein Gegengewicht oder eine mechanische Feder vorgesehen sind.

5. Vorrichtung nach Anspruch 1, **dadurch gekennzeichnet, dass** als Mittel zur Stabilisierung der Orthese (6) ein an ihr befestigter Stab (48) vorgesehen ist, welcher in einem Führungsrohr (46) geführt wird, welches wiederum an der Decke fahrbar befestigt ist, wobei zur Vor-, Rück- und Seitwärtsstabilisierung eine Rollenführung mit Rollen (55a - 55d, 56a - 56d) vorgesehen ist, die in Führungsschienen (50a, 50b) geführt wird.

6. Vorrichtung nach einem der Ansprüche 1 - 5, **dadurch gekennzeichnet, dass** die Hüftorthese in ihrer Breite verstellbar ist.

7. Vorrichtung nach einem der Ansprüche 1 - 6, **dadurch gekennzeichnet, dass** die Beinteile (80a, 80b) aus Beinschienen (63a - 66a, 63b - 66b) bestehen, die ineinander verschiebbar sind, wodurch die Beinteile (80a, 80b) in der Länge verstellbar sind.

8. Vorrichtung nach einem der Ansprüche 1 - 7, **dadurch gekennzeichnet, dass** die Beinteile (80a, 80b) Manschetten (72a, 72b, 73a, 73b, 74a, 74b) aufweisen, die stufenlos 'anterior-posterior' und 'medial-lateral' verstellt werden können.

9. Vorrichtung nach einem der Ansprüche 1 - 8, **dadurch gekennzeichnet, dass** die Manschetten (72a, 72b, 73a, 73b, 74a, 74b) aus einem halbrunden Bügel (126) und einem Band (131) bestehen und dass das Band (131) so am Bügel (126) befestigt ist, dass es sich frei um eine Drehachse im Zentrum des Beines des Patienten drehen lässt.

10. Vorrichtung nach Anspruch 9, **dadurch gekennzeichnet, dass** die verschiedenen Einstellungen der Orthese (6) wie Hüftbreite, Beinlängen und Manschettenpositionen durch Markierungen (70, 71, 91, 92, 124, 125) gekennzeichnet sind.

11. Vorrichtung nach einem der Ansprüche 1 - 10, **dadurch gekennzeichnet, dass** für die Regelung der Antriebe (75a, 75b, 76a, 76b) der Orthese (6) eine Regeleinheit (141) vorgesehen ist, deren Eingangsgrössen Benutzerdaten (142), deren Ausgangsgrössen Steuersignale (143a, 143b) für die Orthese und das Laufband und deren Regelgrösse Messgrössen (144) sind.

12. Vorrichtung nach einem der Ansprüche 1 - 11, **dadurch gekennzeichnet, dass** als Knieantriebe (75a, 75b) und Hüftantriebe (76a, 76b) je ein Kugelgewindespindelantrieb vorgesehen ist.

13. Verfahren zum Betrieb einer Vorrichtung nach einem der Ansprüche 1 - 12, **dadurch gekennzeichnet, dass** die Orthese (6) vom Laufband (1) weggedreht wird, um dem Patienten den Zugang zum Laufband (1) zu ermöglichen, dass die Orthese (6) über dem Laufband (1) positioniert und am Patienten fixiert wird, wobei die Orthese (6) mittels einem Entlastungsmechanismus entlastet wird, und dass die Orthese (6) angetrieben und geregelt und das Laufband (1) angetrieben und gesteuert wird.

14. Verfahren nach Anspruch 13, **dadurch gekennzeichnet, dass** das Parallelogramm (5) mit der Orthese (6) am Geländer (3) des Laufbandes (1) so gelagert wird, dass es sich nach hinten öffnen lässt, wonach der Patient mit dem Rollstuhl auf das Laufband (1) gefahren wird, dass der Patient im Laufbandgurt (16) befestigt, bzw. über dem Laufband (1) aufgehängt wird, und dass danach die Orthese (6) von hinten am Parallelogramm (5) auf das Laufband (1) gedreht und dem hängenden Patienten angezogen wird.

15. Verfahren nach Anspruch 13 oder 14, **dadurch gekennzeichnet, dass** die Antriebe (75a, 75b, 76a, 76b) der Orthese (6) von einer Regeleinheit (141) so geregelt werden, dass sich die Beine des Patienten in einem natürlichen, physiologischen Gangmuster auf dem Laufband (1) bewegen, wobei die zur Erzeugung der physiologischen Bewegungsabläufe benötigten Sollkurven (142d) von der Regeleinheit (141) aufgrund der eingegebenen patientenspezifischen Einstellungen (142c) und der jeweiligen Messgrössen (144) angepasst werden.

16. Verfahren nach einem der Ansprüche 13 - 15, **dadurch gekennzeichnet, dass** die Bewegungen der Orthese (6) mit der Laufbandgeschwindigkeit synchronisiert werden.

17. Verfahren nach einem der Ansprüche 13 - 16, **dadurch gekennzeichnet, dass** die Regeleinheit (141) die Bewegung der Beine mit der Geschwindigkeit des Laufbandes (1) synchronisiert, bzw. ihr anpasst, indem eine Triggereinheit (148) durch ein Triggersignal (149) den Beginn einer Standphase und somit den zeitlichen Verlauf des Bewegungsablaufes signalisiert, und die Sollkurven (142d) dementsprechend angepasst als Steuersignale (143a) an die Antriebe (75a, 75b, 76a, 76b) der Orthese (6) ausgegeben werden.

18. Verfahren nach einem der Ansprüche 13 - 17, **dadurch gekennzeichnet, dass** die Einstellungen der verstellbaren Orthese (6) an den Markierungen (70, 71, 91, 92, 124, 125) abgelesen, gespeichert und rekonstruiert werden.

## Claims

1. Apparatus for treadmill training of walking-disabled patients, comprising a treadmill, a relief mechanism for the patient and a driven orthotic device, **characterized in that** means for stabilizing the orthotic device (6) are provided that prevent the patient from tipping forward, backward and sideward, that the orthotic device (6) consists of a hip orthotic device and two leg parts (80a, 80b), whereby two hip drives (76a, 76b) are provided for moving the hip orthotic device and two knee drives (75a, 75b) are provided for moving the leg parts, that the hip orthotic device and the leg parts (80a, 80b) are adjustable, whereby the leg parts (80a, 80b) are provided with cuffs (72a, 72b, 73a, 73b, 74a, 74b) which are adjustable in size and position, and that a control unit (141) is provided for controlling the movements of the orthotic device (6) and controlling the speed of the treadmill (1).

2. Apparatus as claimed in claim 1, **characterized in that** a parallelogram (5) that is fixed in a height-adjustable manner on the railing (3) of the treadmill (1) is provided as means for stabilizing the orthotic device (6).

3. Apparatus as claimed in claim 2, **characterized in that** the parallelogram (5) consists of a base frame (20), an orthotic device part (21) and two carriers (22a, 22b) that are interconnected via bearings (23a-23d), that on the base frame (20), on the one lower side, a first bearing element (27) is attached, with which the base frame (20) or the parallelogram (5), respectively, are positioned in a rotatable manner and are fixed on the first rail (3) of the treadmill (1) in a height-adjustable manner, that on the base frame (20), on the other lower side, a second bearing element (28) that can be flipped open and closed is attached, with which the base frame (20) or the parallelogram (5), respectively, can be locked to the second rail (3) of the treadmill (1) after the completed rotating movement around the first bearing element (27), and that an orthotic device holder (24) that is provided with means for attaching the orthotic device (6) is attached to the orthotic device part (21).

4. Apparatus as claimed in claim 2 or 3, **characterized in that** a relief mechanism is attached to the parallelogram (5) for compensating the weight of the orthotic device (6), whereby preferably a gas pressure spring (29), a counter-weight or a mechanical spring is provided for this purpose.

5. Apparatus as claimed in claim 1, **characterized in that** as a means for stabilizing the orthotic device (6), a rod (48) that is attached to it has been provided, said rod being guided in a guide pipe (46) which again is attached in a drivable manner to the ceiling, whereby a roller guide with rollers (55a - 55d, 56a - 56d) that is guided in guide tracks (50a, 50b) has been provided for forward, backward and sideward stabilization.

6. Apparatus as claimed in one of claims 1 to 5, **characterized in that** the hip orthotic device is adjustable in its width.

7. Apparatus as claimed in one of claims 1 to 6, **characterized in that** the leg parts (80a, 80b) consist of leg braces (63a - 66a, 63a - 66b) that can be moved inside each other so that the leg parts (80a, 80b) are adjustable in length.

8. Apparatus as claimed in one of claims 1 to 7, **characterized in that** the leg parts (80a, 80b) are provided with cuffs (72a, 72b, 73a, 73b, 74a, 74b) that can be adjusted continuously "anterior-posterior" and "médial-lateral".

9. Apparatus as claimed in one of claims 1 to 8, **characterized in that** the cuffs (72a, 72b, 73a, 73b, 74a, 74b) consist of a semi-round hoop (126) and a tape (131) and that the tape (131) is attached to the hoop (126) in such a way that in can be freely wound around a rotary axis in the centre of a patient's leg.

10. Apparatus as claimed in claim 9, **characterized in that** the different settings of the orthotic device (6) such as hip width, the leg lengths and cuff positions are marked with marks (70, 71, 91, 92, 124, 125).

11. Apparatus as claimed in one of claims 1 to 10, **characterized in that** a control unit (141) is provided for controlling the drives (75a, 75b, 76a, 76b) of the orthotic device (6), the input values of said unit being user data (142), the output values being control signals (143a, 143b) for the orthotic device and the treadmill and its control values being measuring values (144).

12. Apparatus as claimed in one of claims 1 to 11, **characterized in that** a ball screw spindle drive is provided for each the knee drive (75a, 75b) and the hip drive (76a, 76b).

13. Method for operating the apparatus as claimed in one of claims 1 to 12, **characterized in that** the orthotic device (6) is turned away from the treadmill (1) in order to permit the patient to gain access to the treadmill (1), that the orthotic device (6) is positioned above the treadmill (1) and is fixed to the patient, the orthotic device (6) being relieved by a relief mechanism, and that the orthotic device (6) is driven and controlled and the treadmill (1) is driven and controlled.

14. Method as claimed in claim 13, **characterized in that** the parallelogram (5) is mounted with the orthotic device (6) at the railing (3) of the treadmill (1) in such a way that in can be opened towards the back, whereupon the patient is driven in the wheel chair onto the treadmill (1), and **in that** the patient is secured in the treadmill belt (16) or hung above the treadmill (1), and **in that** then the orthotic device (6) is rotated from the back at the parallelogram (5) onto the treadmill (1) and is tightened on the suspended patient.

15. Method as claimed in claim 13 or 14, **characterized in that** the drives (75a, 75b, 76a, 76b) of the orthotic device (6) are controlled by a control unit (141) in such a way that the legs of the patient are moved in a natural, physiological walking pattern on the treadmill (1), whereby the desired curves (142d) necessary for creating the physiological sequences of movement are adapted by the control unit (141) based on the entered patient-specific settings (142c) and respective measuring values (144).

16. Method as claimed in one of claims 13 to 15, **characterized in that** the movements of the orthotic device (6) are synchronized with the treadmill speed.

17. Method as claimed in one of claims 13 to 16, **characterized in that** the control unit (141) synchronizes the movement of the legs with or adapts it to the speed of the treadmill (1) **in that** a trigger unit (148) signals the beginning of a standing phase and thus the course of a sequence of movements over time with a trigger signal (149), and the desired curves (142d) are output to the drives (75a, 75b, 76a, 76b) of the orthotic device (6), adapted appropriately as control signals (143a).

18. Method as claimed in one of claims 13 to 17, **characterized in that** the settings of the adjustable orthotic device (6) are read at the markings (70, 71, 91, 92, 124, 125), are stored and reconstructed.

## Revendications

1. Dispositif pour l'entraînement sur tapis roulant de patients handicapés de la marche, comprenant un tapis roulant, un mécanisme de déchargement pour le patient et une orthèse entrainée, **caractérisé en ce que** des moyens de stabilisation de l'orthèse (6) sont prévus, lesquels empêchent un basculement du patient vers l'avant, vers l'arrière et latéralement, **en ce que** l'orthèse (6) se compose d'une orthèse de la hanche et de deux parties pour les jambes (80a, 80b), deux entraînements des hanches (76a, 76b) étant prévus pour le déplacement de l'orthèse de la hanche et deux entraînements des genoux (75a, 75b) étant-prévus pour le déplacement des parties pour les jambes, l'orthèse de la hanche et les parties pour les jambes (80a, 80b) étant réglables, les parties pour les jambes (80a, 80b) présentant des manchettes (72a, 72b, 73a, 73b, 74a, 74b) de dimension et de position réglables, et **en ce qu'**une unité de réglage (141) est prévue pour le réglage des mouvements de l'orthèse (6) et pour le contrôle de la vitesse du tapis roulant (1).

2. Dispositif selon la revendication 1, **caractérisé en ce que** l'on prévoit en tant que moyen de stabilisation de l'orthèse (6) un parallélogramme (5) qui est fixé sur la balustrade (3) du tapis roulant (1) de manière réglable en hauteur.

3. Dispositif selon la revendication 2, **caractérisé en ce que** le parallélogramme (5) se compose d'un bâti de base (20), d'une partie côté orthèse (21) et de deux supports (22a, 22b) qui sont connectés l'un à l'autre par le biais de paliers (23a-23d), **en ce qu'**un premier élément de palier (27) est monté sur le bâti de base (20) sur un côté inférieur, avec lequel le bâti de base (20), respectivement le parallélogramme (5), sont montés à pivotement et sont fixés de manière réglable en hauteur sur la première balustrade (3) du tapis roulant (1), **en ce qu'**un deuxième élément de palier (28) rabattable et verrouillable est monté sur le bâti de base (20) sur l'autre côté inférieur, avec lequel le bâti de base (20), respectivement le parallélogramme (5), peuvent être bloqués après avoir effectué le mouvement de rotation autour du premier élément de palier (27) sur la deuxième balustrade (3) du tapis roulant (1), et **en ce qu'**un support d'orthèse (24) est monté sur la partie côté orthèse (21), lequel présente des moyens pour la fixation de l'orthèse (6).

4. Dispositif selon la revendication 2 ou 3, **caractérisé en ce qu'**un mécanisme de décharge est monté sur le parallélogramme (5) en vue de compenser le poids de l'orthèse (6), un ressort à pression pneumatique (29), un contre-poids ou un ressort mécanique étant prévus de préférence à cet effet.

5. Dispositif selon la revendication 1, **caractérisé en ce que** l'on prévoit, en tant que moyen pour stabiliser l'orthèse (6), une barre (48) fixée sur celle-ci, laquelle est guidée dans un tube de guidage (46), lequel est à son tour fixé de manière déplaçable sur le dessus, un guidage à rouleaux avec des rouleaux (55a - 55d, 56a - 56d) étant prévu pour la stabilisation avant, arrière et latérale, lequel est guidé dans des glissières (50a, 50b).

6. Dispositif selon l'une quelconque des revendications 1 à 5, **caractérisé en ce que** l'orthèse pour les hanches est réglable en largeur.

7. Dispositif selon l'une quelconque des revendications 1 à 6, **caractérisé en ce que** les parties pour les jambes (80a, 80b) se composent de rails pour les jambes (63a - 66a, 63a - 66b) qui peuvent coulisser les uns dans les autres, les parties pour les jambes (80a, 80b) étant réglables en longueur.

8. Dispositif selon l'une quelconque des revendications 1 à 7, **caractérisé en ce que** les parties pour les jambes (80a, 80b) présentent des manchettes (72a, 72b, 73a, 73b, 74a, 74b), qui peuvent être réglées progressivement de manière "antérieure-postérieure" et "médiale-latérale".

9. Dispositif selon l'une quelconque des revendications 1 à 8, **caractérisé en ce que** les manchettes (72a, 72b, 73a, 73b, 74a, 74b) se composent d'un arceau semi-circulaire (126) et d'une bande (131) et **en ce que** la bande (131) est fixée sur l'arceau (126) de telle sorte qu'elle puisse tourner librement autour d'un axe de rotation au centre de la jambe du patient.

10. Dispositif selon la revendication 9, **caractérisé en ce que** les divers réglages de l'orthèse (6) tels que la largeur des hanches, la longueur des jambes et les positions des manchettes sont **caractérisés par** des marquages (70, 71, 91, 92, 124, 125).

11. Dispositif selon l'une quelconque des revendications 1 à 10, **caractérisé en ce que** pour le réglage des entraînements (75a, 75b, 76a, 76b) de l'orthèse (6), on prévoit une unité de réglage (141) dont les valeurs d'entrée sont des données d'utilisateur (142), dont les valeurs de sortie sont des signaux de commande (143a, 143b) pour l'orthèse et le tapis roulant et dont les valeurs de réglage sont des valeurs de mesure (144).

12. Dispositif selon l'une quelconque des revendications 1 à 11, **caractérisé en ce que** l'on prévoit en tant qu'entraînements des genoux (75a, 75b) et entraînements des hanches (76a, 76b) à chaque fois un entraînement à vis à billes.

13. Procédé pour faire fonctionner un dispositif selon l'une quelconque des revendications 1 à 12, **caractérisé en ce que** l'orthèse (6) est tournée à l'écart du tapis roulant (1), afin de permettre au patient l'accès au tapis roulant, **en ce que** l'orthèse (6) est positionnée au-dessus du tapis roulant (1) et est fixée au patient, l'orthèse (6) étant déchargée au moyen d'un mécanisme de déchargement, et **en ce que** l'orthèse (6) est entraînée et réglée et le tapis roulant (1) est entraîné et commandé.

14. Procédé selon la revendication 13, **caractérisé en ce que** le parallélogramme (5) est monté avec l'orthèse (6) sur la balustrade (3) du tapis roulant (1) de telle sorte qu'il puisse s'ouvrir vers l'arrière, après quoi le patient est amené avec sa chaise roulante sur le tapis roulant (1), **en ce que** le patient est fixé dans la sangle du tapis roulant (16), ou est suspendu au-dessus du tapis roulant (1), et **en ce qu'**ensuite, l'orthèse (6) est tournée depuis l'arrière du parallélogramme (5) sur le tapis roulant (1) et est amenée au patient suspendu.

15. Procédé selon la revendication 13 ou 14, **caractérisé en ce que** les entraînements (75a, 75b, 76a, 76b) de l'orthèse (6) sont réglés par une unité de réglage (141), **en ce que** les jambes du patient sont déplacées suivant un modèle de marche physiologique naturelle sur le tapis roulant (1), les courbes de consigne (142d) nécessaires pour la réalisation des déroulements des mouvements physiologiques étant adaptées par l'unité de réglage (141) sur la base des réglages (142c) spécifiques au patient indiqués et des valeurs de mesure respectives (144).

16. Procédé selon l'une quelconque des revendications 13 à 15, **caractérisé en ce que** les mouvements de l'orthèse (6) sont synchronisés avec la vitesse du tapis roulant.

17. Procédé selon l'une quelconque des revendications 13 à 16, **caractérisé en ce que** l'unité de réglage (141) synchronise le mouvement des jambes avec la vitesse du tapis roulant (1) ou l'adapte à celle-ci, **en ce qu'**une unité de déclenchement (148) signale, par un signal de déclenchement (149), le début d'une phase de position debout et donc l'allure temporelle du déroulement du mouvement, et les courbes de consigne (142d) sont adaptées en conséquence sous forme de signaux de commande (143a) aux entraînements (75a, 75b, 76a, 76b) de l'orthèse (6).

18. Procédé selon l'une quelconque des revendications 13 à 17, **caractérisé en ce que** les réglages de l'orthèse réglable (6) sont lus, mémorisés et reconstruits sur les marquages (70, 71, 91, 92, 124, 125).
